(19) Europäisches Patentamt / European Patent Office / Office européen des brevets

(11) **EP 3 949 733 A1**

(12) **EUROPEAN PATENT APPLICATION**

(43) Date of publication:
**09.02.2022 Bulletin 2022/06**

(21) Application number: **20189939.0**

(22) Date of filing: **06.08.2020**

(51) International Patent Classification (IPC):
*A01N 33/12* (2006.01)      *A01N 47/44* (2006.01)
*A01N 31/08* (2006.01)      *A01P 1/00* (2006.01)
*A61L 2/18* (2006.01)

(52) Cooperative Patent Classification (CPC):
(C-Sets available)
**A01N 33/12; A01N 47/44; A61L 2/18**      (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **JVS Products Limited
Chandlers Ford, Hampshire SO53 3TL (GB)**

(72) Inventor: **SCOONES, Robert
Salisbury, Wiltshire SP5 1LE (GB)**

(74) Representative: **Carpmaels & Ransford LLP
One Southampton Row
London WC1B 5HA (GB)**

(54) **DISINFECTANT COMPOSITIONS**

(57)   The present invention relates to compositions which disinfect skin, whilst also being safe to use on skin. The compositions of the invention are also able to disinfect surfaces.

EP 3 949 733 A1

(52) Cooperative Patent Classification (CPC): (Cont.)

C-Sets
**A01N 33/12, A01N 31/08, A01N 47/44;
A01N 47/44, A01N 31/08**

**Description**

**TECHNICAL FIELD**

The present invention relates to compositions which disinfect skin, whilst also being safe to use on skin. The compositions of the invention are also able to disinfect surfaces such as floors, table tops, and kitchen side boards.

**BACKGROUND AND RELATED ART**

Skin and surfaces often come into contact with, provide an environment for and provide a breeding ground for, potentially harmful pathogens. A potentially harmful pathogen is any organism which can cause disease. Non-limiting examples of potentially harmful pathogens include bacteria, viruses, fungi, allergens, moulds and yeasts.

It is common to regularly disinfect an area of human or animal skin to reduce the risk of infection to the human or animal. It is also common to disinfect surfaces. The disinfecting of skin and surfaces in this way is beneficial to human and animal health as it assists in preventing the spread of disease and mitigates the chances of a human or animal contracting a disease by coming into contact with potentially harmful pathogens. Disinfecting an area of skin and/or a surface includes killing or inactivating pathogens on the area of skin and/or the surface, where "killing" means completely or partially killing (i.e. reducing the number of) pathogens on the area of skin and/or surface.

Environments where it is particularly beneficial to disinfect surfaces are hospital and veterinary environments, e.g. in a hospital operating theatre. There are many other environments where it is beneficial to disinfect surfaces including general household cleaning, hospitality venues, hotels, nursing homes, cruise ships, industrial food processing equipment, shops and restaurants. These environments should be disinfected frequently to prevent the build-up of potentially harmful pathogens.

Known disinfectants for skin and/or surfaces include bleach solutions, high-alcohol (i.e. >50%) based disinfectants and general disinfectant liquids such as Dettol™.

However, there are various disadvantages associated with these known disinfectants. Bleach is not suitable for use on skin and therefore does not relate to the same purpose as the present invention. Similarly, disinfectant liquids such as Dettol™ are effective at killing pathogens on surfaces, but most are not safe for application to skin. Further, many pathogens build up resistance to such commonly-used disinfectants.

While high-alcohol based disinfectants have anti-pathogen activity, they are relatively volatile and so evaporate off skin and surfaces in, typically, seconds or less than a minute, which means pathogens are able to build up on the skin/surface almost straight after application of the high-alcohol based disinfectant. Further, high-alcohol based disinfectants can cause skin irritation and dermatitis.

Thus, there is a need for further disinfectants that can be applied safely to skin without causing irritation or dermatitis, and which effectively disinfect skin. There is also a need for such compositions to remain on the skin for an extended time period. It would be desirable if such compositions could also be used to disinfect surfaces such as floors, table tops, and kitchen side boards.

**GENERAL DEFINITIONS**

The term "comprising" encompasses "including" as well as "consisting", e.g. a composition "comprising" X may consist exclusively of X or may include something additional, e.g. X + Y. The term "comprising" used herein also encompasses "consisting essentially of", e.g. a composition "comprising" X may consist of X and any other components that do not materially affect the essential characteristics of the composition.

The term "about" in relation to a numerical value x is optional and means, for example, x + 10%.

**BRIEF DESCRIPTION OF THE FIGURES**

The invention will be described, by way of example only, with reference to the accompanying figures.

Figure 1: MICs for the composition of the invention against various bacteria.

Figure 2: An example of a plaque assay used to show the composition of the invention's viral activity.

The figures are explained further in the "Examples" section.

**DISCLOSURE OF THE INVENTION**

This application has been drafted in sections to aid readability. However, this does not mean that each section is to be read in isolation. To the contrary, unless otherwise specified, each section is to be read with cross-referencing to the other sections, i.e. taking the entire application as a whole. No artificial separation of embodiments is intended, unless explicitly stated. Any of the compositions of the invention described herein may be used for any of the uses described herein.

Where a composition of the invention is said to comprise at least, for example, 99 wt% water, the other components in the composition are to be selected such that they do not exceed 1 wt% in total, i.e. so that the total amount in weight percent in the composition is 100%. Selection of component amounts in this technically sensible way is within the skilled person's skill set.

**SUMMARY OF THE INVENTION**

In a first aspect there is provided a composition comprising: a benzalkonium halide, didecyl dimethyl ammonium halide, chlorhexidine, and p-chloro-m-cresol. These compositions are safe to use as disinfectants on skin - they meet the requirements of European Chemicals Agency Biocidal Product Type 1 (PT1) Directive - and are also highly potent disinfectants (e.g. >5 log reduction against pathogens). The compositions of the invention can also be used to disinfect surfaces - they meet the requirements of European Chemicals Agency Biocidal Product Type 2 (PT2) Directive - and are also highly potent disinfectants (e.g. >5 log reduction against pathogens). Furthermore, the effects of the composition of the invention last for a long time on skin and surfaces when compared with, for example, known high-alcohol based disinfectants, which typically evaporate from skin/a surface in less than a minute. The longer lasting effect of the compositions of the invention is thought to be because the compositions of the invention form a biocidal surface on the skin/surface which remains on the skin/surface for extended time periods.

In a second aspect there is provided a method of disinfecting an area of skin comprising applying the composition of the invention to the skin. As is shown in the examples and described herein, the composition of the invention meet the European Chemicals Agency Biocidal Product Type 1 (PT1) Directive which means, unlike other disinfectant compositions known in the art, it is safe to use on skin.

In a third aspect there is provided a method of disinfecting a surface comprising applying the composition of the invention to the surface.

Other aspects of the invention include a foam, spray, gel, moisturiser and wipe comprising the composition of the invention.

**DETAILED DESCRIPTION OF THE INVENTION**

Compositions of the invention

The compositions of the invention comprise a benzalkonium halide, a didecyl dimethyl ammonium halide; chlorhexidine; and p-chloro-m-cresol.

In an embodiment, the composition of the invention comprises from about 0.01-0.1 wt% of the benzalkonium halide. In an embodiment, the composition of the invention comprises from about 0.01-0.1 wt% of the didecyl dimethyl ammonium halide. In an embodiment, the composition of the invention comprises from about 0.005-0.04 wt% of the chlorhexidine. In an embodiment, the composition of the invention comprises from about 0.0001-0.003 wt% of the p-chloro-m-cresol.

In another embodiment, the composition of the invention comprises at least two of: (i) from about 0.01-0.1 wt% of benzalkonium halide; (ii) from about 0.01-0.1 wt% of didecyl dimethyl ammonium halide; (iii) from about 0.005-0.04 wt% of chlorhexidine; and (iv) from about 0.0001-0.003 wt% of p-chloro-m-cresol.

In another embodiment, the composition of the invention comprises at least three of: (i) from about 0.01-0.1 wt% of benzalkonium halide; (ii) from about 0.01-0.1 wt% of didecyl dimethyl ammonium halide; (iii) from about 0.005-0.04 wt% of chlorhexidine; and (iv) from about 0.0001-0.003 wt% of p-chloro-m-cresol.

In another embodiment, the composition of the invention comprises from about 0.01-0.1 wt% of benzalkonium halide, from about 0.01-0.1 wt% of didecyl dimethyl ammonium halide, from about 0.005-0.04 wt% of chlorhexidine, and from about 0.0001-0.003 wt% of p-chloro-m-cresol.

In another embodiment, the composition comprises from about 0.05-0.1 wt% a benzalkonium halide, and/or from about 0.05-0.1 wt% a didecyl dimethyl ammonium halide, and/or from about 0.02-0.04 wt% chlorhexidine, and/or from about 0.001-0.003 wt% p-chloro-m-cresol.

In a preferred embodiment, the composition comprises from about 0.05-0.1 wt% a benzalkonium halide, from about 0.05-0.1 wt% a didecyl dimethyl ammonium halide, from about 0.02-0.04 wt% chlorhexidine, and from about 0.001-0.003 wt% p-chloro-m-cresol.

In an embodiment, the benzalkonium halide is benzalkonium chloride or benzalkonium bromide. Preferably, the benzalkonium halide is benzalkonium chloride.

In an embodiment, the didecyl dimethyl ammonium halide is didecyl dimethyl ammonium chloride or didecyl dimethyl ammonium bromide. Preferably the didecyl dimethyl ammonium halide is didecyl dimethyl ammonium chloride.

In an embodiment, the composition of the invention comprises one or more alcohols in addition to the benzalkonium halide, didecyl dimethyl ammonium halide, chlorhexidine, and p-chloro-m-cresol. In an embodiment, the one or more alcohols is ethanol. In another embodiment, the one or more alcohols is an alkylene glycol. In another embodiment, the one or more alcohols are ethanol and an alkylene glycol. Suitable alkylene glycols that may be used in the composition of the invention include ethylene glycol, propylene glycol, diethylene glycol, block copolymers of ethyleneoxide and propyleneoxide, any other alkylene glycol formed from combining alkylene oxides or any combination of alkylene glycols. In a preferred embodiment, the alkylene glycol is ethylene glycol.

In an embodiment, the composition comprises 0.05-0.35 wt%, optionally 0.15-0.35 wt%, of ethanol. In another embodiment, the composition comprises 0.01-0.06 wt%, optionally 0.04-0.06 wt%, of an alkylene glycol, which is preferably ethylene glycol. Preferably, the composition comprises ethanol and an alkylene glycol, which is preferably ethylene glycol. More preferably, the composition comprises 0.05-0.35 wt% of ethanol and 0.01-0.06 wt% of ethylene glycol, or 0.15-0.35 wt% of ethanol and 0.04-0.06 wt% of ethylene glycol.

In an embodiment, the composition of the invention comprises a surfactant such as *N,N*-dimethyldodecan-1-amine oxide (lauramine oxide). Preferably, the composition of the invention comprises 0.15-0.2 wt% of the surfactant. In embodiments where the composition of the invention comprises a surfactant, the composition is preferably for use in disinfecting an area of skin, as described herein.

In an embodiment, the composition of the invention comprises water. In an embodiment, the composition comprises at least 90 wt% water, or at least 95 wt% water, or at least 97 wt% water, or at least 99 wt% water, or at least 99.5 wt% water, or at least 99.7 wt% water, or at least 99.8 wt% water. Preferably, the composition comprises at least 99.5 wt% water or at least 99.7 wt% water or at least 99.8 wt% water.

In an embodiment, the composition of the invention comprises less than 99.99 wt% water.

As can be seen from the examples, the compositions of the invention can be used at very low concentrations, i.e. the compositions of the invention comprising at least 90 wt% water or even at least 99.5 wt% water or at least about 99.9 wt% water are potent disinfectants.

In a preferred embodiment, the composition of the invention does not include one or more siloxanes.

In an embodiment, the composition comprises:

a benzalkonium chloride;
a didecyl dimethyl ammonium chloride;
chlorhexidine;
p-chloro-m-cresol;
ethanol; and
ethylene glycol.

In a preferred embodiment, the composition comprises:

a benzalkonium chloride;
a didecyl dimethyl ammonium chloride;
chlorhexidine;
p-chloro-m-cresol;
ethanol;
ethylene glycol; and
water.

In an embodiment, the composition comprises:

1-2 wt% a benzalkonium chloride;
1-2 wt% a didecyl dimethyl ammonium chloride;
0.4-0.9 wt% chlorhexidine;
0.02-0.06 wt% p-chloro-m-cresol;
4-6 wt% ethanol; and
0.5-1.5 wt% ethylene glycol.

In a preferred embodiment, the composition comprises:

1-2 wt% a benzalkonium chloride;
1-2 wt% a didecyl dimethyl ammonium chloride;
0.4-0.9 wt% chlorhexidine;
0.02-0.06 wt% p-chloro-m-cresol;
4-6 wt% ethanol;
0.5-1.5 wt% ethylene glycol, and
the remainder being water.

In a further preferred embodiment, the composition comprises:

1.5 wt% a benzalkonium chloride;
1.5 wt% a didecyl dimethyl ammonium chloride;
0.66 wt% chlorhexidine;
0.04 wt% p-chloro-m-cresol;
4.9 wt% ethanol; and
1 wt% ethylene glycol, and
the remainder being water, i.e. 90.4 wt% water.

In an embodiment, the composition comprises:

0.05-0.1 wt% a benzalkonium chloride;
0.05-0.1 wt% a didecyl dimethyl ammonium chloride;
0.02-0.04 wt% chlorhexidine;
0.001-0.003 wt% p-chloro-m-cresol;
0.15-0.35 wt% ethanol; and
0.04-0.06 wt% ethylene glycol.

In a preferred embodiment, the composition comprises:

0.05-0.1 wt% a benzalkonium chloride;
0.05-0.1 wt% a didecyl dimethyl ammonium chloride;
0.02-0.04 wt% chlorhexidine;
0.001-0.003 wt% p-chloro-m-cresol;
0.15-0.35 wt% ethanol;
0.04-0.06 wt% ethylene glycol; and
the remainder being water.

In a further preferred embodiment, the composition comprises:

0.075 wt% a benzalkonium chloride;
0.075 wt% a didecyl dimethyl ammonium chloride;
0.033 wt% chlorhexidine;
0.002 wt% p-chloro-m-cresol;
0.245 wt% ethanol;
0.05 wt% ethylene glycol; and
the remainder being water, i.e. 99.52 wt% water.

In an embodiment, the composition comprises:

0.01-0.1 wt% a benzalkonium chloride;
0.01-0.1 wt% a didecyl dimethyl ammonium chloride;
0.005-0.04 wt% chlorhexidine;
0.0001-0.003 wt% p-chloro-m-cresol;
0.05-0.35 wt% ethanol; and
0.010-0.06 wt% ethylene glycol.

In a preferred embodiment, the composition comprises:

0.01-0.1 wt% a benzalkonium chloride;
0.01-0.1 wt% a didecyl dimethyl ammonium chloride;
0.005-0.04 wt% chlorhexidine;
0.0001-0.003 wt% p-chloro-m-cresol;
0.05-0.35 wt% ethanol;
0.010-0.06 wt% ethylene glycol; and
the remainder being water.

In a further preferred embodiment, the composition comprises:

0.023 wt% a benzalkonium chloride;
0.023 wt% a didecyl dimethyl ammonium chloride;
0.01 wt% chlorhexidine;
0.0006 wt% p-chloro-m-cresol;
0.074 wt% ethanol;
0.015 wt% ethylene glycol, and
the remainder being water, i.e. 99.86 wt% water.

The composition of the invention may be formulated in any way that is suitable for application to an area of skin and/or a surface. When the composition is for use in disinfecting an area of skin, the composition is preferably formulated as a foam, moisturiser or gel, in a wipe, as a shampoo, as a facial cleanser, or as a body wash. When the composition of the invention is formulated as a shampoo, the composition preferably comprises a surfactant.

When the composition is for use in disinfecting a surface, the composition is preferably formulated in a wipe or as a liquid for use as a spray.

Uses of the compositions of the invention

The compositions of the invention can be used to disinfect an area of skin, i.e. in methods of disinfecting an area of skin, where the method comprises applying the composition to the skin. In an embodiment, the composition of the invention is used in a method of killing or inactivating one or more pathogens on an area of skin, the method comprising applying the composition to the skin.

As is shown in the examples, the compositions of the invention are highly potent (e.g. > log 5 reduction) against a range of pathogens. Moreover, the compositions of the invention are safe to use on skin - they are non-irritating and meet the requirements of the European Chemicals Agency Biocidal Product Type 1 (PT1) Directive, which is a directive for biocidal products used for human hygiene purposes applied on or in contact with human skin or scalps for the primary purpose of disinfecting the skin.

The compositions of the invention can also be used to disinfect a surface such as a table top or floor, i.e. in methods of disinfecting a surface, where the method comprises applying the composition to the surface. The surface can be made from any material, for example, metal, plastic, glass, stone, enamel, ceramic or wood. In an embodiment, the composition of the invention is used in a method of killing or inactivating one or more pathogens on a surface, the method comprising applying the composition to the surface.

The compositions of the invention also meet the requirements of the European Chemicals Agency Biocidal Product Type 2 (PT2) Directive, which is a directive for disinfectants and algaecides for application to surfaces, but not intended for direct application to humans or animals.

In an embodiment, the one or more pathogens on the area of skin or surface is one or more bacteria, i.e. the composition of the invention may be used in a method of killing or inactivating bacteria on an area of skin or a surface (Example 1). In a preferred embodiment, the one or more bacteria is/are rod shaped and/or spherical shaped. In another preferred embodiment, the one or more bacteria is/are *Pseudomonas aeruginosa, Staphylococcus aureus, Enterococcus hirae,* Methicillin-resistant *Staphylococcus aureus* (MRSA), *Salmonella* Typhimurium, *Klebsiella pneumoniae, Escherichia coli* and/or *Listeria monocytogenes.*

In an embodiment, the one or more pathogens on the area of skin or surface is one or more virus, i.e. the composition of the invention may be used in a method of killing or inactivating one or more virus on an area of skin or a surface. In an embodiment, the virus is an enveloped virus. Examples of enveloped viruses include: DNA viruses such as herpesviruses, poxviruses, hepadnaviruses, and asfarviridae; RNA viruses such as flavivirus, alphavirus, togavirus, coronavirus, hepatitis D, orthomyxovirus, paramyxovirus, rhabdovirus, bunyavirus, and filovirus; and retroviruses. In another embodiment, the enveloped virus is a DNA virus. In another embodiment, the enveloped virus is an RNA virus.

In a preferred embodiment, the virus is a coronavirus which is preferably SARS-CoV-2. In another preferred embodiment, the virus is a poxvirus which is preferably vaccinia virus, e.g. a modified vaccinia virus such as modified vaccinia virus ankara (MVA), ATCC VR-1508. The composition of the invention shows high antiviral activity against viruses, even when used at low concentrations, as can be seen from Example 2. The inventor hypothesised that owing to the high bactericidal activity (>log 5 reduction) in relation to a variety of Gram-positive and Gram-negative bacteria (Example 1) the composition of the invention would show antiviral activity (such as activity against coronavirus).

In an embodiment, the composition of the invention may be applied to the skin or surface before the skin or surface is exposed to the pathogen, e.g. the virus. In a preferred embodiment, the composition of the invention is applied to the skin or surface at least 30 seconds, more preferably at least 1 minute, before the skin or surface is exposed to the virus. As can be seen from Example 2(a), when the composition of the invention was applied to a clean surface (i.e. containing no virus) for, e.g., 1 minute, and then the SARS-CoV-2 virus was applied to the surface, no virus was found subsequently on the surface.

In an embodiment, the methods described herein, i.e. the methods of disinfecting an area of skin, are not methods for treatment on the human or animal body by therapy or surgery.

## EXAMPLES

All of the examples provided herein involved experiments which were performed under non-disclosure agreements.

### Example 1: Gram-positive and Gram-neaative bactericidal activity

The "composition of the invention" used in this example (i.e. Examples 1A-1D) had the following components:

0.075 wt% a benzalkonium chloride;
0.075 wt% a didecyl dimethyl ammonium chloride;
0.033 wt% chlorhexidine;
0.002 wt% p-chloro-m-cresol;
0.245 wt% ethanol;
0.05 wt% ethylene glycol; and
99.52 wt% water.

Example 1A: Bactericidal activity according to EN 13727:2012+A2:2015

Tests 1 and 2 below were carried out by Abbott Analytical Ltd. under a non-disclosure agreement to evaluate the activity of the composition of the invention against a range of bacteria under dirty conditions (defined below).

*(i) Test 1*

*Experimental conditions*

- Method = Dilution-neutralisation
- Neutraliser = 30 g/l Polysorbate 80 + 3 g/l Lecithin + 1 g/l L-histidine + 1 g/l L-cysteine
- Product diluent = sterile hard water (300 mg/L $CaCO_3$)
- Contact time = 5 minutes $\pm$ 10 seconds
- Test temperature = 20 $\pm$ 1 °C.
- Interfering substance = 3.0 g/l bovine albumin + 3.0 ml/l sheep erythrocytes ("dirty conditions")
- Temperature of incubation = 36 °C $\pm$ 1 °C

*Results*

| Bacterial strain | lg reduction |
|---|---|
| *Pseudomonas aeruginosa* (DSM 939) | > 5.09 |

(continued)

| Bacterial strain | Ig reduction |
|---|---|
| *Staphylococcus aureus* (NCTC 10788) | > 5.14 |
| *Eneterococcus hirae* (DSM 3320) | > 5.35 |

*(ii) Test 2*

*Experimental conditions*

- Method = Dilution-neutralisation

- Neutraliser = 100.0 g/l Polysorbate 80 + 30.0 g/l Lecithin + 30.0 g/L Tryptone Soya Broth + 5.0 g/l Sodium thiosulphate +1.0 g/l L-histidine (Neutraliser B)

- Product diluent = hard water

- Contact time = 5 minutes ± 10 seconds

- Test temperature = 20 ± 1 °C.

- Interfering substance = 3.0 g/l bovine albumin + 3.0 ml/l sheep erythrocytes ("dirty conditions")

- Temperature of incubation = 36 °C ± 1 °C

*Results*

| Bacterial strain | Ig reduction |
|---|---|
| Methicillin-resistant *Staphylococcus aureus* (MRSA) | > 5.31 |
| *Escherichai coli* (NCTC 10418) | > 5.27 |
| *Pseudomonas aeruginosa* (NCIMB 10421)) | > 5.10 |

*Conclusions*

The composition of the invention shows bactericidal activity according to EN 13727:2012+A2:2015 against a range of bacteria after at least 5 minutes at 20 °C under dirty conditions. The results show that the composition of the invention has >5 Ig reduction against the bacteria even at very low concentrations, i.e. even when the composition contains at least 99.5 wt% water.

Example 1B: Bactericidal activity according to EN 14561:2006

Tests were carried out by Abbott Analytical Ltd. under a non-disclosure agreement to evaluate the activity of the composition of the invention against a range of bacteria under dirty conditions (defined below).

*Experimental conditions*

- Method = Dilution-neutralisation
- Neutraliser = 30 g/l Polysorbate 80 + 3 g/l Lecithin + 1 g/l L-histidine + 1 g/l L-cysteine

- Product diluent = sterile hard water (300 mg/L CaCO$_3$)
- Contact time = 10 minutes $\pm$ 10 seconds
- Test temperature = 20 $\pm$ 1 °C.
- Interfering substance = 3.0 g/l bovine albumin + 3.0 ml/l sheep erythrocytes ("dirty conditions")
- Temperature of incubation = 36 °C $\pm$ 1 °C

*Results*

| Bacterial strain | lg reduction |
|---|---|
| *Pseudomonas aeruginosa* (DSM 939) | > 5.18 |
| *Staphylococcus aureus* (NCTC 10788) | > 5.28 |
| *Eneterococcus hirae* (DSM 3320) | > 5.17 |

*Conclusions*

The composition of the invention shows bactericidal activity according to EN 13727:2012+A2:2015 against a range of bacteria after at least 10 minutes at 20 °C under dirty conditions. The results show that the composition of the invention has >5 lg reduction against the bacteria even at very low concentrations, i.e. even when the composition contains at least 99.5 wt% water.

Example 1C: Bactericidal activity according to EN 1276:2019

Tests were carried out by Abbott Analytical Ltd. under a non-disclosure agreement to evaluate the activity of the composition of the invention against a range of bacteria under dirty conditions (defined below).

*Experimental conditions*

- Method = Dilution-neutralisation (pour plate)
- Neutraliser = 100.0 g/l Polysorbate 80 + 30.0 g/l Lecithin + 30.0 g/L Tryptone Soya Broth + 5.0 g/l Sodium thiosulphate +1.0 g/l L-histidine (Neutraliser B)
- Neutraliser validation = Validated in accordance with EN 1276:2019 (5.5.2)
- Product diluent = hard water
- Contact time = 5 minutes $\pm$ 10 seconds
- Test temperature = 20 $\pm$ 1 °C.
- Interfering substance = 3.0 g/l bovine albumin ("dirty conditions")
- Temperature of incubation = 36 °C $\pm$ 1 °C

*Results*

| Bacterial strain | lg reduction |
|---|---|
| *Salmonella* Typhimurium | > 5.32 |
| *Listeria monocytogenes* | > 5.34 |

*Conclusions*

The composition of the invention shows bactericidal activity according to EN 1276:2019 against a range of bacteria after at least 5 minutes at 20 °C under dirty conditions. The results show that the composition of the invention has >5 lg reduction against the bacteria even at very low concentrations, i.e. even when the composition contains at least 99.5 wt% water.

Example 1D: MIC assay for bacteria exposed to the composition of the invention

*Materials and methods*

• Bacterial strains

Bacterial strains were supplied by NCTC (PHE, UK). The Gram-positive multidrug resistant *Staphylococcus aureus* (MRSA) (NCTC 13143), the Gram-negative multidrug resistant *Klebsiella pneumoniae* (NDM-1) (NCTC 13443), the Gram-negative *Pseudomonas aeruginosa* (PA01) (NCTC 10332), the Gram-positive *Listeria monocytogenes* (NCTC 13372) and the Gram-negative *Escherichia coli* O157:H7 (NCTC 12900) were grown as overnight cultures in Mueller Hinton Broth (MHB).

• Minimum Inhibitory Concentration (MIC) assay for bacteria exposed to the composition of the invention

The composition of the invention was serially diluted two-fold in MHB to create a concentration range from 0.1% (v/v) to $9.8 \times 10^{-5}$% (v/v) in individual wells of a 96-well plate. Two separate batches of the composition of the invention were tested to compare the efficacy levels. $5 \times 10^5$ colony forming units (CFUs) of bacteria from overnight cultures were added to each well, which were then incubated for 24 hours at 37°C. Tests were conducted in triplicate. Wells containing MHB with no composition of the invention were used as positive controls. Wells containing only MHB were used as contamination controls. After incubation the turbidities of the wells were examined by eye. For each dilution series the well that contained the lowest concentration of the composition of the invention while still demonstrating no turbidity was identified as the MIC for that test condition.

*Results*

• General

Log reduction in infectious bacteria was calculated according to the following formula:

Log reduction = log10 (plaque forming unit (PFU) before treatment) minus log10 (PFU after treatment)
1-log reduction = 90% reduction
2-log reduction = 99% reduction
3-log reduction = 99.9% reduction
4-log reduction = 99.99% reduction

• Bacteria MIC assay

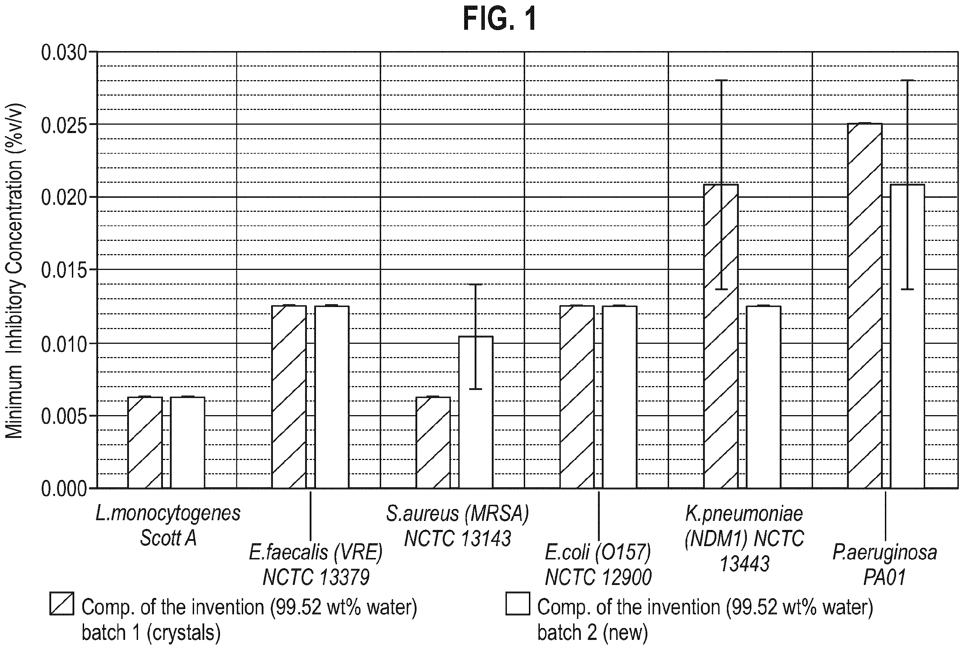

MIC assays were performed with both batches of the composition of the invention. The composition of the invention demonstrated a very high efficacy level for the bacterial strains tested with MIC values ranging from 0.006% (v/v) to 0.025% (v/v) (Figure 1). This variation is expected, as bacteria are known to demonstrate varying susceptibilities to antimicrobial compounds.

**Example 2: Viral activity**

**2(a) Coronavirus - SARS-CoV-2**

The following two compositions of the invention were used in this example.
"Composition of the invention (99.52 wt% water)" had the following components:

0.075 wt% a benzalkonium chloride;
0.075 wt% a didecyl dimethyl ammonium chloride;
0.033 wt% chlorhexidine;
0.002 wt% p-chloro-m-cresol;
0.245 wt% ethanol;
0.05 wt% ethylene glycol; and
99.52 wt% water.

"Composition of the invention (99.86 wt% water)" had the following components:

0.023 wt% a benzalkonium chloride;
0.023 wt% a didecyl dimethyl ammonium chloride;
0.010 wt% chlorhexidine;
0.0006 wt% p-chloro-m-cresol;
0.074 wt% ethanol;
0.015 wt% ethylene glycol, and
99.86 wt% water.

Materials and methods

• Viral strains and cell lines

Human coronavirus SARS-CoV-2 and a kidney cell line, VERO-E6, were supplied by Public Health England (PHE), UK. Cells were maintained in minimal essential medium (MEM) supplemented with GlutaMax-1, nonessential amino acids, and 5% foetal calf serum and incubated at 37°C and 5% $CO_2$. Cells were passaged twice a week using trypsin (0.25%)-EDTA and were not used beyond passage 30 (P30) (which occurred before the onset of senescence, but susceptibility to infection diminished greatly from P30). Viral stocks were prepared by infecting cells at multiplicity of infection of 0.01 for 4 to 7 days until a significant cytopathic effect (CPE) was observed. Infected cell supernatant was stored at -80°C.

• Preparation of sample surfaces

Stainless steel coupons (10 by 10 by 0.5 mm) were degreased in acetone, stored in absolute ethanol, and flamed prior to use.

• Infectivity assay for SARS-CoV-2 exposed to surfaces

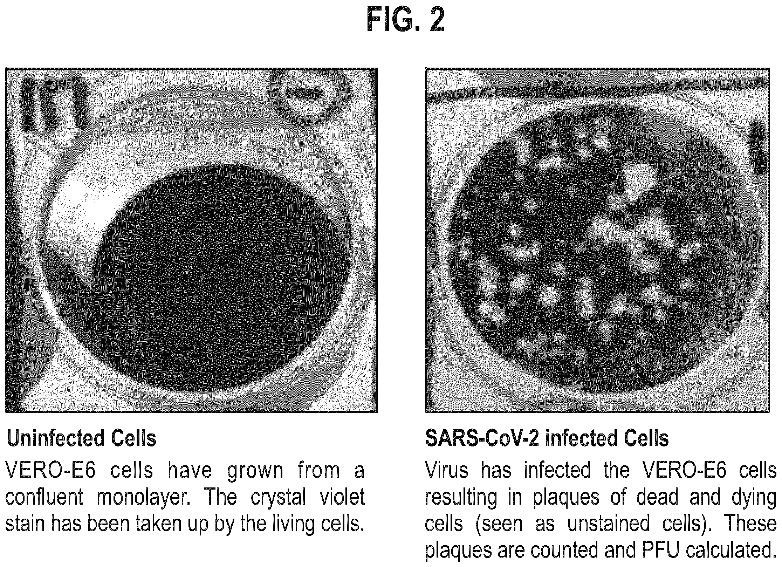

The virus was applied to a surface and allowed to dry for 1 hour before application of the composition of the invention.
Infected cell supernatant preparations of SARS-CoV-2 were spread over coupons of the test surface and incubated at room temperature. The virus was removed from the coupons after various time points up to 60 mins using infection medium and assayed for infectious virus by a plaque assay. Dilutions were prepared in infection medium, and 400 μl aliquots were plated onto confluent monolayers of VERO-E6 cells that had been prepared 24 h earlier in 12-well plates. The inoculum was removed after 60 min and replaced with Avicel overlays, and plates were incubated at 37°C and 5% $CO_2$ for 3 days. The monolayers were fixed for 30 minutes in 8% paraformaldehyde, stained with crystal violet and plaques in the monolayer enumerated. An example of a plaque assay is shown in Figure 2.

Results

• General

Log reduction in infectious virus was calculated according to the following formula:

Log reduction = log10 (PFU before treatment) minus log10 (PFU after treatment)
1-log reduction = 90% reduction
2-log reduction = 99% reduction
3-log reduction = 99.9% reduction
4-log reduction = 99.99% reduction

1 μl or 10 μl SARS-CoV-2 stock (2.0 x 106/ml) was added to stainless steel coupons and allowed to dry. 1 μl or 2 μl of the composition of the invention was added and left for between 30 seconds and 10 minutes. The virus was recovered into 1 ml infection medium, after different exposure times to the composition of the invention and enumerated by plaque assay.

• Test 1

(a) 1 µl of SARS-CoV-2 virus was dried on stainless steel coupons, then 1 µl of the composition of the invention (99.52 wt% water) was applied for 30 seconds, 5 minutes or 10 minutes. In a parallel arm, the virus was also washed off the surface without the composition of the invention (99.52 wt% water) being applied to the surface, to confirm recovery of the virus by this method. The composition of the invention (99.52 wt% water) prevented virus transmission from SARS-CoV-2 spiked surface after only 30 seconds contact time.

| Test surface | PFU/surface | Log reduction |
|---|---|---|
| Virus on stainless steel coupon | 2000 | 0 |
| Virus, then composition of the invention (99.52 wt% water) for 30 seconds | 0 | 3.3* |
| Virus, then composition of the invention (99.52 wt% water) for 1 minute | 0 | 3.3* |
| Virus, then composition of the invention (99.52 wt% water) for 5 minutes | 0 | 3.3* |

*3.3 is the maximum log-reduction available for this assay, equivalent to no infectious virus remaining.

(b) Stainless steel coupons were pre-treated with 1 µl of the composition of the invention (99.52 wt% water), left to dry, then 1 µl SARS-CoV-2 was applied to the surface before pipetting it off the surface and washing using infection medium.

After 1 minute and 5 minutes on stainless steel coupons pre-treated with the composition of the invention (99.52 wt% water), no infectivity was detected. These 1 µl spikes had the time to dry out, depositing all virions on the pre-treated surface.
The composition of the invention (99.52 wt% water) alone at that dilution (1 µl of solution in 1 ml infection medium giving a final concentration 0.005%) was not cytotoxic to Vero-E6 cells.

| Test surface | PFU/surface | Log reduction |
|---|---|---|
| Virus on stainless steel coupon | 2000 | 0 |
| Composition of the invention (99.52 wt% water) for 30 seconds, then virus | 900 | 0.346 |
| Composition of the invention (99.52 wt% water) for 1 minute, then virus | 0 | 3.3* |
| Composition of the invention (99.52 wt% water) for 5 minutes, then virus | 0 | 3.3* |

*3.3 is the maximum log-reduction available for this assay, equivalent to no infectious virus remaining.

• Test 2

10 µl (20000 virions) of SARS-CoV-2 virus was dried on stainless steel coupons, then 2 µl of the composition of the invention (99.86 wt% water) was applied for 30 seconds, 1 minute, 2 minutes, 5 minutes or 10 minutes. The composition of the invention (99.86 wt% water) spread over the surface of the coupon covering all the dried virus.
The results below show that the composition of the invention (99.86 wt% water) prevented virus transmission from SARS-CoV-2 spiked surface after only 30 seconds contact time.

| Test surface | PFU/surface | Log reduction |
|---|---|---|
| Virus, then composition of the invention (99.86 wt% water) for 30 seconds | 0 | 4.3[ϕ] |
| Virus then composition of the invention (99.86 wt% water) for 1 minute | 0 | 4.3[ϕ] |
| Virus, then composition of the invention (99.86 wt% water) for 2 minutes | 0 | 4.3[ϕ] |
| Virus, then composition of the invention (99.86 wt% water) for 5 minutes | 0 | 4.3[ϕ] |
| Virus, then composition of the invention (99.86 wt% water) for 10 minutes | 0 | 4.3[ϕ] |

[ϕ]4.3 is the maximum log-reduction available for this assay, equivalent to no infectious virus remaining.

Summary

This example shows that the composition of the invention is very effective at inactivating SARS-CoV-2 coronavirus contamination of stainless steel surfaces (within as little as 30 seconds). In addition, when the composition of the invention is pre-coated onto a surface and allowed to dry, before adding SARS-CoV-2, the virus is inactivated after 30 seconds to 1 minute. This means a contaminated surface could be cleaned with the composition of the invention which would eradicate any existing coronavirus contamination and any subsequent contamination would continue to be inactivated. In effect this means a non-biocidal surface can become temporarily converted to a biocidal one. This is especially important with respiratory diseases where disease symptoms result in regular contamination of surfaces from coughs, sneezes, contaminated hands etc. and the infectious dose is low representing a significant infection risk, especially if the contamination occurs between cleaning regimes.

This example also shows that the composition of the invention is able to inactivate coronaviruses (such as SARS-CoV-2) within a short time-frame (e.g. 30 seconds), even when used at extremely low concentrations.

**2(b) Enveloped viruses - BS EN 14476**

The following two compositions of the invention were used in this example.

"Composition of the invention (99.52 wt% water)" had the following components:

0.075 wt% a benzalkonium chloride;
0.075 wt% a didecyl dimethyl ammonium chloride;
0.033 wt% chlorhexidine;
0.002 wt% p-chloro-m-cresol;
0.245 wt% ethanol;
0.05 wt% ethylene glycol; and
99.52 wt% water.

"Composition of the invention (99.14 wt% water)" had the following components:

0.135 wt% a benzalkonium chloride;
0.135 wt% a didecyl dimethyl ammonium chloride;
0.059 wt% chlorhexidine;
0.0036 wt% p-chloro-m-cresol;
0.44 wt% ethanol; and
0.09 wt% ethylene glycol, and
99.14 wt% water.

Scope

The standard method BS EN 14476 describes a test method and the minimum requirements for virucidal activity of a chemical disinfectant and antiseptic products that form a homogenous physically stable preparation when diluted with hard water, or (in the case of ready to use products that are not diluted when applied) with water. This European Standard applies to products that are used in the medical area in the fields of hygienic handrub, hygienic handwash, instrument disinfection by immersion, surface disinfection by wiping, spraying, flooding or other means and textile disinfection.

This European standard applies to areas and situations where disinfection is medically indicated (e.g. hospitals).

Outline of test method (obligatory test conditions)

A sample of the composition of the invention is diluted in synthetic hard water (in products diluted at point of use) or (in the case of ready to use products) water is added to a test suspension of vacciniavirus in a solution of interfering substance. The mixture is maintained at one of the temperatures and contact times specified in the standard. At the end of this contact time, an aliquot is taken; the virucidal action in this portion is immediately suppressed by a validated method (dilutions of the sample in ice-cold cell maintenance medium). The dilutions are transferred into cell culture units either using monolayer or cell suspension. Infectivity tests are done either by plaque test or quantal tests. After incubation, the titres of infectivity are calculated according to Spearman and Käber or by plaque counting. Reduction of vacciniavirus infectivity is calculated from differences of log virus titres before (virus control) and after treatment with the composition of the invention.

Acceptance criteria

At least a 4 $\log_{10}$ reduction against the test virus is required. The test is deemed valid where all control requirements are met.

Test information

| | |
|---|---|
| Neutralisation Method: | Dilution |
| Product Diluent: | Distilled water |
| Experimental Conditions: | Clean |
| Interfering Substance: | Clean 0.3g/l Bovine Albumin |
| Test Temperature: | 20°C $\pm$ 1°C |
| Temperature of Incubation: | 37°C $\pm$1 °C |
| Identification of the Viral Strains: | Modified vaccinia virus Ankara (MVA), ATCC VR-1508 (enveloped claim only) |
| Contact Times: | 2 minutes + 10s |

Deviations from the standard method

None

Results

| Composition of invention | Contact time | log TCID50 | log reduction | Pass/Fail |
|---|---|---|---|---|
| 99.14 wt% water | 2 mins | 3.42 | 4.58 | Pass |
| 99.52 wt% water | 2 mins | 4.00 | 4.00 | Pass |

Conclusion

This example shows that the compositions of the invention have high viral activity, even when used at low concentrations. Specifically, this test shows that the compositions of the invention meet the criteria for enveloped viruses as detailed in EN14476:2013+A2:2019.

## Example 3: Skin irritation tests

The "composition of the invention" used in this example had the following components:

0.075 wt% a benzalkonium chloride;
0.075 wt% a didecyl dimethyl ammonium chloride;
0.033 wt% chlorhexidine;
0.002 wt% p-chloro-m-cresol;
0.245 wt% ethanol;
0.05 wt% ethylene glycol; and
99.52 wt% water.

An *in vitro* evaluation of the irritancy potential of the composition of the invention using a tissue engineered human skin model was carried out by Dr David Voegeli, Associate professor of Nursing, at the Skin Health Research Group, under a non-disclosure agreement.

The overall aim of the project was to investigate the effects of several compositions of the invention on an approved tissue engineered human skin model (The European Union Reference Laboratory for alternatives to animal testing, EURL-ECVAM).

The objectives were as follows:

1. To determine the degree of skin irritancy caused by a range of compositions of the invention.

2. To determine *in vitro* toxicity of a range of compositions of the invention to human keratinocytes.

Study Protocol

*Day 0*

A 24 tissue insert EpiDerm™ skin irritation kit was purchased from MatTek In Vitro Life Science Laboratories, Slovakia (EPI-200-SIT). On receipt of the kit, the tissues were visually assessed for integrity and conditioned by transferring into sterile 6 well plates, adding 0.9 ml medium and incubating for 60 minutes at 37 °C, 5% $CO_2$, 95% RH. After 60 minutes, the tissues were removed, placed in fresh medium and further incubated for 18 hours.

Prior to experimental testing, all compositions to be tested were assessed to ensure they would not react with the cell viability assay used and cause any artificial colour changes. No abnormal reactions were noted.

*Day 1*

The following formulations plus positive control (5% SDS) and negative control (PBS) were tested in duplicate or triplicate:

| Formulation | Number of tissues |
| --- | --- |
| Positive control - 5% SDS | 3 |
| Negative control - PBS | 3 |
| Composition of invention | 3 |
| Composition of invention (6.25 wt% in moisturiser) | 2 |
| Composition of invention (6.25 wt% in gel) | 2 |
| Composition of invention (6.25 wt% in Foam) | 2 |

To test for irritancy potential, 30 µl of liquid formulation, plus positive and negative control or 25 mg semisolid (gel / moisturiser) were applied to surface of each test tissue insert. After dosing the inserts were incubated for 35 minutes (37 °C, 5% $CO_2$, 95% RH). Inserts were then removed from the incubator and allowed to rest for a further 25 minutes at room temperature within a sterile laminar flow hood. Once each tissue had been exposed to the test substance for a total of 60 minutes, each insert was rinsed 15 times using sterile DPBS to remove the test substance. Finally, each insert was submerged three times in 150 ml sterile DPBS, blotted dry and the tissue surface gently dried using a sterile cotton swab. The inserts were placed back into fresh 6 well plates with 0.9 ml medium and returned to the incubator for 24 hours.

*Day 2*

After incubating for 24 hours, inserts were placed in fresh medium (0.9 ml) and the medium from the 24 hour incubation saved and stored at -80 °C for later analysis, if required.

*Day 3*

Fresh MTT assay solution was prepared by diluting MTT concentrate with diluent to achieve a final concentration of 1 mg/ml. 300 $\mu$l of MTT solution was added to each well of a 24 well plate and a single tissue insert transferred to each well. Once complete, the plate was then incubated for 3 hours (37 °C, 5% $CO_2$, 95% RH).

After 3 hours the MTT solution was aspirated from each well, and the wells rinsed twice with DPBS. The tissue inserts were then transferred to a fresh 24 well plate and each insert immersed by the addition of 2 ml isopropanol. The plate was then covered to prevent evaporation of the isopropanol and placed on a plate shaker for 2 hours to allow formazan extraction.

Once extraction was complete, 200 $\mu$l samples from each tissue were added to a 96 well plate in duplicate for the MTT assay of cell viability, and 200 $\mu$l isopropanol to 4 wells as blanks. The optical density of each well was read using a spectrophotometric plate reader at 570 nm without using a reference filter.

Data analysis

The mean OD (optical density) from the blank wells was subtracted from the OD of each well. The mean OD from each duplicate was then obtained and the individual relative tissue viability for each tissue treated with a test substance (TS), the positive control (PC) and the negative control (NC) calculated according to the following formulas:

$$\text{Relative viability TS (\%)} = [\text{ODTS} / \text{Mean of ODNC}] \times 100$$

$$\text{Relative viability NC (\%)} = [\text{ODNC} / \text{mean of ODNC}] \times 100$$

$$\text{Relative viability PC (\%)} = [\text{ODPC} / \text{mean of ODNC}] \times 100$$

Relative cell viability was calculated for each test tissue as a percentage of the mean of the negative control tissues. According to the EU and GHS classification (R38/ Category 2 or no label), an irritant is predicted if the mean relative tissue viability of three individual tissues exposed to the test substance is reduced below 50% of the mean viability of the negative controls. Therefore the skin irritation potential of the product tested was confirmed if the remaining relative cell viability (RV%) was below 50% and potential not to be irritant if above 50%.

Results

Macroscopic and microscopic examination of inserts:
All tissues received were intact and viable on visual inspection and were used. Following dosing of the tissues a random selection of inserts were examined by low powered light microscopy to ensure no damage had been caused during the procedure. No cell damage was observed in any of the inserts examined.

MTT assay

The raw OD of each well at 570 nm is shown below (Table 1):

|   | 1 | 2 | 3 | 4 | 5 | 6 | 7 | 8 | 9 | 10 | 11 | 12 |
|---|---|---|---|---|---|---|---|---|---|---|---|---|
| A | 0.08 | 0.17 | 0.19 | 0.15 | 1.05 | 2.23 | 1.52 | 1.62 | 1.57 | 0.14 | 1.66 | 1.34 |
| B | 0.08 | 0.18 | 0.19 | 0.15 | 1.07 | 2.26 | 1.52 | 1.65 | 1.57 | 0.14 | 1.70 | 1.32 |
| C | 0.08 | 0.18 | 0.19 | 0.15 | 1.08 | 2.26 | 1.53 | 1.64 | 1.58 | 0.14 | 2.09 | 1.32 |
| D | 3.16 | 3.06 | 2.15 | 3.05 | 1.83 | 3.09 | 3.29 | 3.16 | 3.12 | 3.06 | 3.16 | 3.08 |
| E | 3.20 | 3.11 | 2.18 | 3.07 | 1.81 | 3.11 | 3.33 | 3.19 | 3.15 | 3.11 | 3.19 | 3.09 |
| F | 3.11 | 3.02 | 2.16 | 3.02 | 1.82 | 3.05 | 3.21 | 3.09 | 3.07 | 3.01 | 3.09 | 3.03 |
| G | 0.04 | 0.04 | 0.04 | 0.05 | 0.05 | 0.05 | 0.05 | 0.05 | 0.05 | 0.05 | 0.05 | 0.05 |
| H | 0.05 | 0.05 | 0.05 | 0.05 | 0.05 | 0.05 | 0.05 | 0.05 | 0.05 | 0.05 | 0.05 | 0.05 |

Key: **Positive control**, Negative control, Test composition, blank

Table 1

Relative Viability

Calculation of the relative viability for the controls and compositions tested is shown below in Table 2.

Table 2: Relative viability

| Formulation | Mean RV% |
|---|---|
| Positive control - 5% SDS | 9.0 |
| Negative control - PBS | 100 |
| Composition of invention | 94.6 |
| Composition of invention (6.25 wt% in moisturiser) | 148.9 |
| Composition of invention (6.25 wt% in gel) | 195.9 |
| Composition of invention (6.25 wt% in foam) | 189.4 |

Conclusions

This study was designed to investigate the potential skin irritant effects of a range of compositions of the invention intended for application to skin. A total of four products were tested, plus positive control and negative control. Calculation of RV% as a measure of irritancy showed all compositions according to the invention tested had an RV% over 50, with the majority (75%) having an RV% above 100.

Overall, these data demonstrate that none of the tested compositions would be likely to cause skin irritation, which is a requirement in the European Chemicals Agency Biocidal Product Type 1 (PT1) Directive.

Also described herein are the following clauses:

1. A composition comprising:

   a benzalkonium halide;
   a didecyl dimethyl ammonium halide;
   chlorhexidine; and
   p-chloro-m-cresol.

2. The composition according to clause 1, wherein the composition comprises:

   0.01-0.1 wt% a benzalkonium halide;
   0.01-0.1 wt% a didecyl dimethyl ammonium halide;
   0.005-0.04 wt% chlorhexidine; and
   0.0001-0.003 wt% p-chloro-m-cresol.

3. The composition according to clause 2, wherein the composition comprises:

0.05-0.1 wt% a benzalkonium halide;
0.05-0.1 wt% a didecyl dimethyl ammonium halide;
0.02-0.04 wt% chlorhexidine; and
0.001-0.003 wt% p-chloro-m-cresol.

4. The composition according to any one of the preceding clauses, wherein the benzalkonium halide is benzalkonium chloride.

5. The composition according to any one of the preceding clauses, wherein the didecyl dimethyl ammonium halide is didecyl dimethyl ammonium chloride.

6. The composition according to any one of the preceding clauses, wherein the composition comprises one or more additional alcohols.

7. The composition according to clause 6, wherein the composition comprises ethanol.

8. The composition according to clause 7, wherein the composition comprises 0.05-0.35 wt% of the ethanol.

9. The composition according to clause 8, wherein the composition comprises 0.15-0.35 wt% of the ethanol.

10. The composition according to any one of clauses 6-9, wherein the composition comprises an alkylene glycol.

11. The composition according to clause 10, wherein the alkylene glycol is ethylene glycol.

12. The composition according to clause 10 or clause 11, wherein the composition comprises 0.01-0.06 wt% of the alkylene glycol.

13. The composition according to clause 12, wherein the composition comprises 0.04-0.06 wt% of the alkylene glycol.

14. The composition according to any one of the preceding clauses, wherein the composition comprises a surfactant, optionally wherein the surfactant is N,N-dimethyldodecan-1-amine oxide.

15. The composition according to any one of the preceding clauses, wherein the composition comprises water.

16. The composition according to clause 15, wherein the composition comprises at least 90 wt% water.

17. The composition according to clause 16, wherein the composition comprises at least 99 wt% water.

18. The composition according to clause 17, wherein the composition comprises at least 99.5 wt% water.

19. The composition according to any one of clauses 1, 2, 4-8, 10-12 and 14, when not dependent directly or indirectly on clauses 3, 9 and/or 13, wherein the composition comprises at least 99.8 wt% water.

20. The composition according to any one of clauses 1-18, wherein the composition comprises:

0.05-0.1 wt% a benzalkonium chloride;
0.05-0.1 wt% a didecyl dimethyl ammonium chloride;
0.02-0.04 wt% chlorhexidine;
0.001-0.003 wt% p-chloro-m-cresol;
0.15-0.35 wt% ethanol; and
0.04-0.06 wt% ethylene glycol.

21. The composition according to clause 20, where in the composition comprises:

0.05-0.1 wt% a benzalkonium chloride;
0.05-0.1 wt% a didecyl dimethyl ammonium chloride;
0.02-0.04 wt% chlorhexidine;
0.001-0.003 wt% p-chloro-m-cresol;

0.15-0.35 wt% ethanol;
0.04-0.06 wt% ethylene glycol; and
the remainder being water.

22. The composition according to clause 21, where in the composition comprises:

0.075 wt% a benzalkonium chloride;
0.075 wt% a didecyl dimethyl ammonium chloride;
0.033 wt% chlorhexidine;
0.002 wt% p-chloro-m-cresol;
0.245 wt% ethanol;
0.05 wt% ethylene glycol; and
99.52 wt% water.

23. The composition according to any one of clauses 1, 2, 4-8, 10-12 and 14-19, when not dependent directly or indirectly on clauses 3, 9 and/or 13, wherein the composition comprises:

0.01-0.1 wt% a benzalkonium chloride;
0.01-0.1 wt% a didecyl dimethyl ammonium chloride;
0.005-0.04 wt% chlorhexidine;
0.0001-0.003 wt% p-chloro-m-cresol;
0.05-0.35 wt% ethanol; and
0.010-0.06 wt% ethylene glycol.

24. The composition according to clause 23, wherein the composition comprises:

0.01-0.1 wt% a benzalkonium chloride;
0.01-0.1 wt% a didecyl dimethyl ammonium chloride;
0.005-0.04 wt% chlorhexidine;
0.0001-0.003 wt% p-chloro-m-cresol;
0.05-0.35 wt% ethanol;
0.010-0.06 wt% ethylene glycol; and
the remainder being water.

25. The composition according to clause 24, wherein the composition comprises:

0.023 wt% a benzalkonium chloride;
0.023 wt% a didecyl dimethyl ammonium chloride;
0.010 wt% chlorhexidine;
0.0006 wt% p-chloro-m-cresol;
0.074 wt% ethanol;
0.015 wt% ethylene glycol; and
99.86 wt% water.

26. The composition according to any one of clauses 1, 4-7, 10, 11, 14-16, when not dependent directly or indirectly on clauses 2, 3, 8, 9, 12 and/or 13, wherein in the composition comprises:

1-2 wt% a benzalkonium chloride;
1-2 wt% a didecyl dimethyl ammonium chloride;
0.4-0.9 wt% chlorhexidine;
0.02-0.06 wt% p-chloro-m-cresol;
4-6 wt% ethanol; and
0.5-1.5 wt% ethylene glycol, and
the remainder being water.

27. The composition according to clause 26, where in the composition comprises:

1.5 wt% a benzalkonium chloride;

1.5 wt% a didecyl dimethyl ammonium chloride;
0.66 wt% chlorhexidine;
0.04 wt% p-chloro-m-cresol;
4.9 wt% ethanol; and
1 wt% ethylene glycol, and
90.4 wt% water.

28. A method of disinfecting an area of skin, the method comprising applying the composition according to any one of the preceding clauses to the area of skin.

29. A method of disinfecting a surface, the method comprising applying a composition according to any one of clauses 1-27 to the surface.

30. A method of killing or inactivating one or more pathogens on an area of skin, the method comprising applying the composition according to any one of clauses 1-27 to the area of skin.

31. A method of killing or inactivating one or more pathogens on a surface, the method comprising applying a composition according to any one of clauses 1-27 to the surface.

32. The method according to clause 30 or clause 31, wherein the one or more pathogens is one or more bacteria.

33. The method according to clause 32, wherein the one or more bacteria is/are rod shaped and/or spherical shaped.

34. The method according to clause 32 or 33, wherein the one or more bacteria is/are *Pseudomonas aeruginosa, Staphylococcus aureus, Enterococcus hirae,* Methicillin-resistant *Staphylococcus aureus* (MRSA), *Salmonella* Typhimurium, *Klebsiella pneumoniae, Escherichia coli* and/or *Listeria monocytogenes.*

35. The method according to clause 30 or clause 31, wherein the one or more pathogens is one or more virus.

36. The method according to clause 35, wherein the one or more virus is an enveloped virus.

37. The method according to clause 35 or 36, wherein the virus is a coronavirus which is preferably SARS-CoV-2.

38. The method according to clause 35 or clause 36, wherein the virus is a poxvirus which is preferably a vaccinia virus, more preferably wherein the vaccinia virus is a modified vaccinia virus such as modified vaccinia virus ankara (MVA), ATCC VR-1508.

39. The method according to any one of claims 28-38, wherein the composition is applied to the surface or skin before the surface or skin is exposed to a pathogen.

40. The method according to claim 39, wherein the composition is applied to the surface or skin at least 30 seconds, preferably at least 1 minute, before the surface or skin is exposed to the pathogen.

41. The method according to any one of clauses 28-40, wherein the method is not a method for treatment on the human or animal body by therapy or surgery.

42. A foam for application to skin, the foam comprising the composition according to any one of clauses 1-27.

43. A spray for application to skin, the spray comprising the composition according to any one of clauses 1-27.

44. A gel for application to skin, the gel comprising the composition according to any one of clauses 1-27.

45. A wipe for application to skin and/or a surface, the wipe comprising the composition according to any one of clauses 1-27.

46. The method according to any one of clauses 28-41, wherein the composition is applied to the area of skin and/or surface using the foam, spray, gel, moisturiser or wipe as defined in clause 42-45, respectively.

**Claims**

1. A composition comprising:

   a benzalkonium halide;
   a didecyl dimethyl ammonium halide;
   chlorhexidine; and
   p-chloro-m-cresol.

2. The composition according to claim 1, wherein the composition comprises:

   0.01-0.1 wt% a benzalkonium halide;
   0.01-0.1 wt% a didecyl dimethyl ammonium halide;
   0.005-0.04 wt% chlorhexidine; and
   0.0001-0.003 wt% p-chloro-m-cresol.

3. The composition according to claim 1 or claim 2, wherein the benzalkonium halide is benzalkonium chloride, and/or wherein the didecyl dimethyl ammonium halide is didecyl dimethyl ammonium chloride.

4. The composition according to any one of the preceding claims, wherein the composition comprises one or more additional alcohols which is optionally ethanol, optionally wherein the composition comprises 0.05-0.35 wt% of the ethanol.

5. The composition according to any one of the preceding claims, wherein the composition comprises an alkylene glycol, optionally wherein the alkylene glycol is ethylene glycol, optionally wherein the composition comprises 0.01-0.06 wt% of the alkylene glycol.

6. The composition according to any one of the preceding claims, wherein the composition comprises water.

7. The composition according to claim 6, wherein the composition comprises at least 90 wt% water.

8. The composition according to claim 7, wherein the composition comprises at least 99.5 wt% water, optionally at least 99.8 wt% water.

9. The composition according to claim 8, where in the composition comprises:

   0.01-0.1 wt% a benzalkonium chloride;
   0.01-0.1 wt% a didecyl dimethyl ammonium chloride;
   0.005-0.04 wt% chlorhexidine;
   0.0001-0.003 wt% p-chloro-m-cresol;
   0.05-0.35 wt% ethanol;
   0.01-0.06 wt% ethylene glycol; and
   the remainder being water.

10. The composition according to any one of claims 1-7, where in the composition comprises:

    1-2 wt% a benzalkonium chloride;
    1-2 wt% a didecyl dimethyl ammonium chloride;
    0.4-0.9 wt% chlorhexidine;
    0.02-0.06 wt% p-chloro-m-cresol;
    4-6 wt% ethanol; and
    0.5-1.5 wt% ethylene glycol, and
    the remainder being water.

11. A method of disinfecting an area of skin and/or a surface, the method comprising applying the composition according to any one of the preceding claims to area of the skin and/or surface.

12. A method of killing or inactivating one or more pathogens on an area of skin and/or a surface, the method comprising applying the composition according to any one of claims 1-10 to the area of the skin and/or surface.

13. The method according to claim 12, wherein the one or more pathogens is one or more bacteria, optionally wherein the one or more bacteria is/are *Pseudomonas aeruginosa, Staphylococcus aureus, Enterococcus hirae,* Methicillin-resistant *Staphylococcus aureus* (MRSA), *Salmonella typhimurium, Klebsiella pneumoniae, Escherichia coli* and/or *Listeria monocytogenes.*

14. The method according to claim 12, wherein the one or more pathogens is one or more virus, optionally wherein the one or more virus is an enveloped virus, optionally wherein (i) the virus is a coronavirus which is preferably SARS-CoV-2, and/or (ii) the virus is a poxvirus which is preferably vacciniavirus.

15. A foam, spray, gel, moisturiser or wipe for application to skin, the foam, spray, gel or wipe comprising the composition according to any one of claims 1-10.

FIG. 1

# FIG. 2

**Uninfected Cells**

VERO-E6 cells have grown from a confluent monolayer. The crystal violet stain has been taken up by the living cells.

**SARS-CoV-2 infected Cells**

Virus has infected the VERO-E6 cells resulting in plaques of dead and dying cells (seen as unstained cells). These plaques are counted and PFU calculated.

Europäisches
Patentamt

European
Patent Office

Office européen
des brevets

# EUROPEAN SEARCH REPORT

Application Number

EP 20 18 9939

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 335 557 A1 (JVS PRODUCTS LTD [GB]) 20 June 2018 (2018-06-20) <br> * abstract * <br> * paragraphs [0001], [0016] - [0031], [0036] * <br> * paragraph [0039] * <br> * claims 1-21 * | 1-15 | INV. <br> A01N33/12 <br> A01N47/44 <br> A01N31/08 <br> A01P1/00 <br> A61L2/18 |
| Y | WO 2012/114062 A1 (MTP INNOVATIONS LTD [GB]; FINAN PETER [GB]) 30 August 2012 (2012-08-30) <br> * page 1, lines 3-6 * <br> * page 9, lines 13-29; example 1 * <br> * page 10, line 29 - page 11, line 8; example 2 * <br> * page 12, line 13 - page 13, line 12 * | 1-15 | |
| Y | WO 2017/222965 A1 (LONZA AG [US]) 28 December 2017 (2017-12-28) <br> * abstract * <br> * paragraphs [0006], [0029], [0058] - [0060] * <br> * Spectradyne; page 31; table 1 * <br> * page 32; tables 4-5 * <br> * claims 1, 3-4 * | 1-15 | TECHNICAL FIELDS SEARCHED (IPC) <br><br> A01N |

-/--

The present search report has been drawn up for all claims

| Place of search | Date of completion of the search | Examiner |
|---|---|---|
| Munich | 8 February 2021 | Hateley, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

Europäisches
Patentamt

European
Patent Office

Office européen
des brevets

# EUROPEAN SEARCH REPORT

Application Number

EP 20 18 9939

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FRANE BANOVIC ET AL: "In vitro comparison of the effectiveness of polihexanide and chlorhexidine against canine isolates of Staphylococcus pseudintermedius , Pseudomonas aeruginosa and Malassezia pachydermatis", VETERINARY DERMATOLOGY, vol. 24, no. 4, 21 June 2013 (2013-06-21), pages 409-e89, XP55129333, ISSN: 0959-4493, DOI: 10.1111/vde.12048 * abstract * * page 412, right-hand column, lines 20-27 * | 1-15 | |

-----

TECHNICAL FIELDS
SEARCHED     (IPC)

The present search report has been drawn up for all claims

| Place of search | Date of completion of the search | Examiner |
|---|---|---|
| Munich | 8 February 2021 | Hateley, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 18 9939

This annex lists the patent family members relating to the patent documents cited in  the above-mentioned European search report.
The members are as contained in the European Patent Office EDP file on
The European Patent Office is in no way liable for these particulars which are merely given for the purpose of information.

08-02-2021

| Patent document cited in search report | | Publication date | Patent family member(s) | | Publication date |
|---|---|---|---|---|---|
| EP 3335557 | A1 | 20-06-2018 | EP | 3041352 A1 | 13-07-2016 |
| | | | EP | 3335557 A1 | 20-06-2018 |
| | | | ES | 2655263 T3 | 19-02-2018 |
| | | | ES | 2790403 T3 | 27-10-2020 |
| | | | GB | 2517778 A | 04-03-2015 |
| | | | NO | 3041352 T3 | 07-04-2018 |
| | | | SG | 11201707118U A | 30-10-2017 |
| | | | US | 2016212994 A1 | 28-07-2016 |
| | | | US | 2018160682 A1 | 14-06-2018 |
| | | | WO | 2015028806 A1 | 05-03-2015 |
| WO 2012114062 | A1 | 30-08-2012 | EP | 2710107 A1 | 26-03-2014 |
| | | | WO | 2012114062 A1 | 30-08-2012 |
| WO 2017222965 | A1 | 28-12-2017 | BR | 112018076819 A2 | 02-04-2019 |
| | | | CN | 109640656 A | 16-04-2019 |
| | | | EP | 3457850 A1 | 27-03-2019 |
| | | | JP | 2019520362 A | 18-07-2019 |
| | | | US | 2020305435 A1 | 01-10-2020 |
| | | | WO | 2017222965 A1 | 28-12-2017 |

EPO FORM P0459

For more details about this annex : see Official Journal of the European Patent Office, No. 12/82